# EUROPEAN PATENT APPLICATION

(11) **EP 4 491 194 A1**
(43) Date of publication of application: **15.01.2025**
(21) Application number: 23767137.5
(22) Date of filing: 07.03.2023
(51) Int. Cl.: A61K 39/12, A61K 38/20, A61K 39/395, A61P 35/00, A61K 39/00

(54) **TRIPLE COMBINATION DRUG DOSING THERAPY FOR HEAD AND NECK CANCER TREATMENT**

(30) Priority: 10.03.2022 KR 20220029827
(71) Applicant: Genexine, Inc., Seoul 07789 (KR); UIF (UNIVERSITY INDUSTRY FOUNDATION), YONSAI UNIVERSITY, Seodaemun-Gu Seoul 03722 (KR)
(72) Inventor: SUNG, Young Chul, Seoul 04419 (KR); WOO, Jung Won, Seoul 06305 (KR); KIM, Hye Ryun, Seoul 03709 (KR); HONG, Min Hee, Seoul 03709 (KR); KIM, Chang Gon, Seoul 06597 (KR)
(74) Representative: Dehns
(86) International application number: PCT/KR2023/003115
(87) International publication number: WO 2023/172036

(57) **Abstract**

The present invention relates to a triple combination drug dosing therapy for head and neck cancer treatment and, more specifically, to a therapeutic method for the treatment of head and neck cancer by dosing an anti-PD1 antibody, a human papilloma virus (HPV) vaccine, and a long-lasting recombinant IL-7 fusion protein. The present invention exhibits a remarkable effect in the treatment of intractable head and neck cancer that has been difficult to treat with existing therapeutic agents.

## Description

### [Technical Field]

The present invention relates to a triple combination drug dosing therapy for head and neck cancer treatment and, more specifically, to a therapeutic method for treating head and neck cancer by administering an anti-programmed death 1 (anti-PD1) antibody, a human papilloma virus (HPV) vaccine, and a long-acting recombinant interleukin-7 (IL-7) fusion protein.

### [Background Art]

Head and neck cancer refers to cancer that occurs in any part of the head and neck except the brain and eyes, and includes oral cancer, pharyngeal cancer (tongue cancer, tonsil cancer), laryngeal cancer, paranasal sinus cancer, salivary gland cancer, and nasopharyngeal cancer. Head and neck cancer has high incidence and mortality rates, with approximately 700,000 new cases and 350,000 deaths occurring worldwide each year, and squamous cell carcinoma of the head and neck accounts for 90% or more.

Various therapeutic methods such as resection, radiotherapy, and chemotherapy have been attempted for the treatment of locally advanced head and neck squamous cell carcinoma (LA HNSCC), but the treatment is difficult because the cancer is often diagnosed at stage 4 when the cancer has already progressed considerably, the pathogenesis is complicated, the stage is difficult to diagnose and predict, and the survival rate remains at 30%.

Recently, the incidence rate of human papilloma virus (HPV)-associated HNSCC has been particularly increasing, and HPV-associated HNSCC mainly originates from the tonsil (base of the tongue). Oropharyngeal cancer (OPC), a clinical subset of HNSCC, is one of the cancers with a particularly high prevalence of HPV-associated tumors, and the incidence rate of HPV-positive OPC is increasing worldwide. HPV-positive OPC has immunological characteristics that are different from those of HPV-negative OPC, is characterized by a high level of T lymphocyte infiltration, and has a low survival rate. In particular, according to a forest plot of the CHECKMATE-141 and KEYNOTE-040 trials, the response rate of HPV-positive HNSCC to anti-programmed cell death-ligand 1 (anti-PD-1)/PD-L1 therapy was lower than expected. In addition, according to the recently published KEYNOTE-412 study results, when a chemoradiotherapy and a maintenance therapy with anti-PD-1 pembrolizumab were assessed with 780 patients with HNSCC, no statistical significance was confirmed compared to the control group. This suggests that HNSCC is still a difficult disease to treat.

Meanwhile, various mechanisms to evade immune surveillance are expressed in tumors induced by HPV. Specifically, a tumor microenvironment (1) downregulates major histocompatibility complex (MHC) Class I expression and at the same time, impairs antigen processing and antigen presentation, (2) induces resistance to immune-mediated cytotoxic T-lymphocyte (CTL) cytotoxicity, (3) expresses immunosuppressive cytokines (e.g., transforming growth factor-β (TGF-β) and IL-10) and immune checkpoint molecules (e.g., PD-L1), and (4) induces immune cells with immunosuppressive properties (e.g., regulatory T cells, myeloid-derived suppressor cells).

HPV DNA vaccine (GX-188E), which preferentially presents HPV antigens to dendritic cells, is known to induce multifunctional T-cell immunity associated with HPV clearance in patients with stage 3 cervical intraepithelial neoplasia, without safety concerns. Furthermore, GX-188E demonstrated remarkable anticancer efficacy (best overall response rate (BORR) 33.3%, progression-free survival (PFS) 2.7 months) and a controllable safety profile in patients with standard-of-care refractory cervical cancer when administered in combination with pembrolizumab (Korean Patent Publication No. 10-2022-0109444).

Meanwhile, GX-I7 is a long-acting IL-7 protein composed of human IL-7 and a hybrid Fc (hyFc) region, which may extend the half-life of IL-7. IL-7 is a potent cytokine required for thymic T-cell development and homeostatic T-cell expansion. For reference, it has been reported that GX-I7 administration may induce T-cell migration to tumor sites along with massive expansion of T-cells (Korean Patent Publication No. 10-2022-0079541). In addition, it was confirmed that thymectomized animals treated with a combination therapy of IL-7 protein and anti-PD-1 antibody exhibited a significant reduction in tumor volume compared to other treatment groups (control, IL-7 alone, and anti-PD-1 antibody alone) (Korean Patent Publication No. 10-2021-0093950).

In the present invention, as the incidence rate of HPV-positive HNSCC has recently increased and the need for customized treatment for HPV-16-positive or HPV-18-positive HNSCC has emerged, a method for promoting tumor-specific immunity and reversing the immunosuppressive tumor microenvironment in HPV-induced tumors was searched for, and it was confirmed that the anti-cancer effect on head and neck cancer, which is known to have a poor prognosis when treated with existing therapeutic methods, is significantly improved through a therapy that administers a combination of an anti-PD1 antibody, an HPV vaccine, and a long-acting recombinant IL-7 fusion protein in a stage before cancer resection for anticancer treatment, and so the present invention was completed.

### [Disclosure]

### [Technical Problem]

The object of the present invention is to provide a pharmaceutical composition for head and neck cancer treatment that exhibits excellent therapeutic effects on intractable head and neck cancer.

### [Technical Solution]

To achieve the above-described object, the present invention provides a pharmaceutical composition for treating head and neck cancer, including an anti-programmed death 1 (anti-PD1) antibody, a human papilloma virus (HPV) vaccine, and a long-acting recombinant interleukin-7 (IL-7) fusion protein.

In the present invention, the anti-PD1 antibody may be selected from the group consisting of pembrolizumab, nivolumab, cemiplimab, pidilizumab, and a combination thereof.

In the present invention, the HPV vaccine may include a polynucleotide encoding HPV-16 and HPV-18 antigens.

In the present invention, the HPV vaccine may be isolated DNA including a polynucleotide encoding the antigens.

In the present invention, the HPV vaccine may include a polynucleotide of SEQ ID NO: 15 or a functional variant having 85% or more sequence identity with SEQ ID NO: 15.

In the present invention, the HPV vaccine may further include a nucleic acid sequence encoding a heterologous polypeptide. In the present invention, the heterologous polypeptide includes an Fms-like tyrosine kinase 3 ligand ("FLT3L") or a part thereof.

In the present invention, the HPV vaccine may further include a nucleotide sequence encoding a signal peptide.

In the present invention, the signal peptide may be selected from a signal peptide of tissue plasminogen activator (tPA), a signal peptide of herpes simplex virus glycoprotein D (HSV gD), a signal peptide of a growth hormone, and any combination thereof.

In the present invention, the long-acting recombinant IL-7 fusion protein may include:
(a) IL-7 or modified IL-7 represented by Chemical Formula (I) below:

   A - IL-7 Chemical Formula (I),

   wherein A is an oligopeptide consisting of one to ten amino acid residues; and
(b) a domain extending the half-life of the IL-7 or the modified IL-7.

In the present invention, the IL-7 may have an amino acid sequence selected from the group consisting of SEQ ID NOs: 16 to 21.

In the present invention, the A may be an oligopeptide consisting of amino acid residues selected from the group consisting of methionine, glycine, or a combination thereof.

In the present invention, the domain may include an Fc region of a modified immunoglobulin.

In the present invention, the long-acting recombinant IL-7 fusion protein may include an amino acid sequence of SEQ ID NO: 39 or a functional variant having 85% or more sequence identity with SEQ ID NO: 39.

In the present invention, the anti-PD-1 antibody may be pembrolizumab or nivolumab, and the single dose is 50 mg to 500 mg.

In the present invention, the anti-PD-1 antibody may be for intravenous inj ection.

In the present invention, the single dose of the HPV vaccine may be 0.5 mg to 5 mg.

In the present invention, the HPV vaccine may be for intramuscular injection.

In the present invention, the single dose of the long-acting recombinant IL-7 fusion protein may be 60 µg/kg to 5,000 µg/kg.

In the present invention, the long-acting recombinant IL-7 fusion protein may be for intramuscular injection.

In the present invention, the anti-PD1 antibody, the HPV vaccine, and the long-acting recombinant IL-7 fusion protein may each be administered once or multiple times.

In the present invention, the anti-PD1 antibody, the HPV vaccine, and the long-acting recombinant IL-7 fusion protein may be administered simultaneously, separately, or sequentially.

In the present invention, the anti-PD1 antibody may be pembrolizumab, and the pembrolizumab may be administered twice, the HPV vaccine may be administered three times, and the long-acting recombinant IL-7 fusion protein may be administered once.

In the present invention, the anti-PD1 antibody may be nivolumab, and the nivolumab may be administered at two-week intervals, the HPV vaccine may be administered eight times, and the long-acting recombinant IL-7 fusion protein may be administered three times.

In the present invention, the pharmaceutical composition may be administered before cancer resection.

In the present invention, the head and neck cancer may be HPV-positive.

In the present invention, the head and neck cancer may be head and neck squamous cell carcinoma.

In the present invention, the head and neck cancer may be oropharyngeal cancer.

In the present invention, the head and neck cancer may be metastatic, recurrent, and/or advanced.

In the present invention, the head and neck cancer may be locally advanced and resectable.

In the present invention, the pharmaceutical composition may be administered to subjects who have not previously received any other anticancer treatment.

In addition, the present invention provides a kit for head and neck cancer treatment, including an anti-PD1 antibody, an HPV vaccine, and a long-acting recombinant IL-7 fusion protein.

In addition, the present invention provides use of a combination of an anti-PD1 antibody, an HPV vaccine, and a long-acting recombinant IL-7 fusion protein in the manufacture of a medicine for treating head and neck cancer.

In addition, the present invention provides a combination of an anti-PD1 antibody, an HPV vaccine, and a long-acting recombinant IL-7 fusion protein for use in the treatment of head and neck cancer.

In addition, the present invention provides a method of treating head and neck cancer, comprising administering an anti-PD1 antibody, an HPV vaccine, and a long-acting recombinant IL-7 fusion protein to a subject in need thereof.

In the present invention, the anti-PD1 antibody, the HPV vaccine, and the long-acting recombinant IL-7 fusion protein may be administered simultaneously, separately, or sequentially.

In the present invention, the anti-PD1 antibody, the HPV vaccine, and the long-acting recombinant IL-7 fusion protein may be administered before cancer resection.

### [Advantageous Effects]

The present invention has the advantage of exhibiting a clinically significantly superior therapeutic effect in intractable head and neck cancer, such as HPV-positive head and neck cancer, which was difficult to treat with conventional standard treatments or surgical treatments alone.

### [Description of Drawings]

FIG. 1 shows a combination drug dosing schedule in a clinical trial according to one embodiment of the present invention.
FIG. 2 shows the results of assessing the pathologic tumor response (%) in a clinical trial according to one embodiment of the present invention.
FIG. 3 shows the results of assessing the radiologic tumor response (%) in a clinical trial according to one embodiment of the present invention.
FIG. 4 shows the results of photographing the head and neck cancer treatment effect in a clinical trial subject according to one embodiment of the present invention.

### [Modes of the Invention]

Unless otherwise defined, all technical and scientific terms used herein have the same meaning as commonly understood by one of ordinary skill in the art to which the present invention pertains. In general, the nomenclature used herein and the experimental methods described below are well known and commonly used in the art.

### Definitions

Unless otherwise mentioned or implied in the context, the following terms and phrases include the meanings given below. Unless explicitly mentioned otherwise or clear from the context, the following terms and phrases do not exclude the meanings that such terms or phrases have acquired in the relevant art. Definitions are provided to aid in describing particular embodiments and are not intended to limit the scope of the subject matter presented herein, because the scope of the subject matter presented herein is limited only by the claims. Unless otherwise defined, all technical and scientific terms used herein have the same meaning as generally understood by one of ordinary skill in the art to which the subject matter provided herein pertains.

The term "pharmaceutical composition" is defined herein to mean a mixture or solution containing at least one therapeutic agent to be administered to a mammal to prevent or treat a particular disease or condition affecting the mammal. In one embodiment, the mammal may be a human.

The term "pharmaceutically acceptable" is defined herein to mean a compound, material, composition, and/or dosage form that is suitable for contact with a tissue of a human patient without excessive toxicity, irritation, allergic reactions, and other problematic complications corresponding to a reasonable benefit/risk ratio within the scope of rational medical judgment.

The term "treating" or "treatment" as used herein includes treatment that relieves, decreases, reduces, or alleviates at least one symptom or delays the progression of a disease in a human patient. For example, treatment may be a reduction of one or more symptoms of a disorder or complete eradication of the disorder. Within the meaning of the present disclosure, the term "treat" also means arresting or delaying the onset of a disease (i.e., the period prior to clinical manifestation of the disease) and/or reducing the risk of developing or worsening the disease. The term "protect" is used herein to mean preventing, delaying, or treating the development or persistence or worsening of a disease in a subject, or all thereof.

The term "pharmaceutically effective amount" or "clinically effective amount" is an amount sufficient to provide observable amelioration in the basic clinically observable signs and symptoms of a disorder being treated.

A "subject" or "individual" or "animal" or "patient" or "mammal" refers to any subject for which diagnosis, prognosis, or treatment is needed, particularly a mammalian subject. A mammalian subject includes humans, livestock, farm animals, zoo animals, sport animals, pets such as dogs, cats, guinea pigs, rabbits, rats, mice, horses, cows, and dairy cows; primates such as apes, monkeys, orangutans, and chimpanzees; canids such as dogs and wolves; felines such as cats, lions, and tigers; equids such as horses, donkeys, and zebras; food animals such as bears, cattle, pigs, and sheep; ungulates such as deer and giraffes; rodents such as mice, rats, hamsters, and guinea pigs; and the like, but is not limited thereto. In a specific embodiment, the mammal is a human subject.

In the present invention, the subject may be a human patient diagnosed with or suffering from head and neck cancer.

The term "combination treatment/therapy," "combined treatment," "combinatorial" or "in combination" refers to treatment using two or more separate therapeutic agents simultaneously and/or at different times within a defined period of time.

The term "about" is used to mean approximately, roughly, around, or in the region of. When the term "about" is used with a numerical range, it modifies the range by extending the boundaries above and below the specified numerical values. Typically, the term "about" is used to modify a specified value up or down (higher or lower) by a variation of 10% to a numerical value above and below.

The term "significant" or "significantly" indicates statistical significance, and it generally refers to a difference of two standard deviations (2SD) or more.

The term "decreased" or "decrease" is used herein to generally refer to a decrease by a statistically significant amount. In some embodiments, "decreased" or "decrease" means a decrease (e.g., to the level of absence or undetectable compared to a reference level) of at least a 10% decrease compared to a reference level, for example, a decrease of at least about 20%, or at least about 30%, or at least about 40%, or at least about 50%, or at least about 60%, or at least about 70%, or at least about 80%, or at least about 90% or more, and a 100% decrease.

The term "increased" or "increase" is used herein to generally refer to an increase by a statistically significant amount. In some embodiments, "increased" or "increase" means an increase of at least 10% compared to a reference level, for example, an increase of at least about 20%, or at least about 30%, or at least about 40%, or at least about 50%, or at least about 60%, or at least about 70%, or at least about 80%, or at least about 90% or more.

The term "comprising" or "comprise" as used herein is used with respect to compositions, methods, and respective component(s) thereof, and may include unspecified elements that are necessary to the method or composition, regardless of the necessity.

The term "consisting of" refers to compositions, methods, and respective components thereof as described herein, excluding any element not mentioned in the description of the relevant embodiment.

Unless otherwise specified, the terms "protein," "polypeptide," and "peptide" may be used interchangeably.

### Anti-PD-1 antibody

Programmed cell death protein 1 (PD-1, also known as CD279) encodes a cell surface membrane protein of an immunoglobulin superfamily expressed on B cells and natural killer (NK) cells. Anti-PD1 antibodies have been used to treat melanoma, non-small cell lung cancer, bladder cancer, prostate cancer, colorectal cancer, head and neck cancer, triple-negative breast cancer, leukemia, lymphoma, and renal cell carcinoma. Exemplary anti-PD1 antibodies include pembrolizumab (KEYTRUDA^{®}, MERCK), nivolumab (BMS-936558, BRISTOL-MYERS SQUIBB), cemiplimab (LIBTAYO^{®}), and pidilizumab (CT-011, CURETECH LTD.).

In one embodiment, the anti-PD1 antibody is pembrolizumab.

Pembrolizumab (KEYTRUDA^{®} (formerly, lambrolizumab)), also known as Merck 3745, MK-3475 or SCH-900475, is a humanized IgG4 monoclonal antibody that binds to PD-1. Pembrolizumab is disclosed in the documents [Hamid, O. et al., New England Journal of Medicine, 2013, 369(2): 134-44]; WO2009/114335; and US 8,354,509.

In another embodiment, the anti-PD1 antibody is nivolumab.

Nivolumab (Opdivo^{®}) is also known as iMDX-1106, MDX-1106-04, ONO-4538, or BMS-936558 and has the Chemical Abstracts Service (CAS) registry number 946414-94-4. Nivolumab is a fully human IgG4 monoclonal antibody that specifically blocks PD-1. Nivolumab (clone 5C4) and other human monoclonal antibodies that specifically bind to PD-1 are disclosed in US 8,008,449 and WO2006/121168.

### Human papilloma virus (HPV) vaccine

An HPV vaccine that may be used as an embodiment of the present invention may preferably include a fusion protein of HPV16 and HPV18 or a polynucleotide encoding the fusion protein.

As one embodiment, the fusion protein includes three or more amino acid sequences selected from the following: (1) an N-terminal part of the E6 protein of HPV16, (2) a C-terminal part of the E6 protein of HPV16, (3) an N-terminal part of the E7 protein of HPV16, (4) a C-terminal part of the E7 protein of HPV16, (5) an N-terminal part of the E6 protein of HPV18, (6) a C-terminal part of the E6 protein of HPV18, (7) an N-terminal part of the E7 protein of HPV18, and (8) a C-terminal portion of the E7 protein of HPV18. Here, the fusion protein does not bind to p53 or form a dimer with the E6 protein of HPV16 or HPV18, and the fusion protein does not bind to pRb or form a dimer with the E7 protein of HPV16 or HPV18. This is described in US 11135262 B2, and the entire contents of US 11135262 B2 are incorporated herein by reference.

Fusion proteins including a fusion polypeptide that modifies the three-dimensional structure of E6 and E7 derived from HPV types 16 and 18 and an immune-enhancing peptide and polynucleotides encoding the same are disclosed in US 9000139 B2. The fusion proteins according to an exemplary embodiment of US 9000139 B2 may include a recombinant fusion polypeptide for modifying the three-dimensional structure of E6 and E7 derived from HPV types 16 and 18. More specifically, the fusion polypeptide is a fusion polypeptide in which amino acids 1 to 85 of the E6 protein, amino acids 1 to 65 of the E7 protein, amino acids 71 to 158 of the E6 protein, and amino acids 51 to 98 of the E7 protein derived from HPV type 16; and amino acids 1 to 85 of the E6 protein, amino acids 1 to 65 of the E7 protein, amino acids 71 to 158 of the E6 protein, and amino acids 51 to 105 of the E7 protein derived from HPV type 18 are linked in sequence. US 9000139 B2 relates to nucleic acid molecules encoding fusion polypeptides and immune-enhancing peptides. Exemplary immune-enhancing peptides include the CD40 ligand, Flt3 ligand, flagellin, and/or OX40. Embodiments of polynucleotides encoding fusion proteins, optimized signal sequences (e.g., tPa), and immune-enhancing peptides (e.g., Flt3 ligand) may be prepared with reference to Example 1 of US 9000139 B2. The entire contents of US 9000139 B2 are incorporated herein by reference.

The term "GX-188E" as used herein refers to a nucleic acid construct having a nucleotide sequence of SEQ ID NO: 15 or a variant thereof having at least about 80% or more, about 81% or more, about 82% or more, about 84% or more, about 85% or more, about 86% or more, about 87% or more, about 88% or more, about 89% or more, about 90% or more, about 91% or more, about 92% or more, about 93% or more, about 94% or more, about 95% or more, about 96% or more, about 97% or more, about 98% or more, about 99% or more, or about 100% sequence identity with SEQ ID NO: 15.

The term "sequence identity" between two polypeptides in the present invention is determined by comparing the amino acid sequence of one polypeptide with the sequence of a second polypeptide. As discussed herein, whether a particular polypeptide is at least about 50%, about 60%, about 70%, about 75%, about 80%, about 85%, about 86%, about 87%, about 88%, about 89%, about 90%, about 91%, about 92%, about 93%, about 94%, about 95%, about 96%, about 97%, about 98%, about 99%, or 100% identical to another polypeptide may be determined using methods and computer programs/software known in the art, such as, but not limited to, the BESTFIT program (Wisconsin Sequence Analysis Package, Version 8 for Unix, Genetics Computer Group, University Research Park, 575 Science Drive, Madison, Wis. 53711). BESTFIT employs the local homology algorithm disclosed in Smith and Waterman, Advances in Applied Mathematics 2:482-489 (1981) to find the best segment of homology between two sequences. When BESTFIT or any other sequence alignment program is used to determine whether a particular sequence is, for example, 95% identical to a reference sequence according to the present invention, parameters are, of course, set such that the percent identity is calculated over the entire length of a reference polypeptide sequence, and a homology gap of up to 5% of the total number of amino acids in the reference sequence is allowed.

An HPV vaccine may be a material described in International Patent Publication Nos. 2016/024255 and 2021/215896, and the entire contents of the literature are incorporated herein by reference.

### Long-acting recombinant IL-7 fusion protein

In a long-acting recombinant IL-7 fusion protein that may be used as one embodiment of the present invention, the IL-7 may be an interleukin-7, a polypeptide that may bind to an IL-7 receptor (also known as CD127) or a polypeptide having the activity of IL-7 or a similar activity thereto.

The term "a polypeptide having the activity of IL-7 or a similar activity thereto" as used herein refers to a polypeptide or protein having the same or similar sequence and activity as IL-7.

The IL-7 herein includes a polypeptide consisting of an amino acid sequence represented by SEQ ID NOs: 16 to 21. In addition, the IL-7 may have about 70%, about 75%, about 80%, about 85%, about 86%, about 87%, about 88%, about 89%, about 90%, about 91%, about 92%, about 93%, about 94%, about 95%, about 96%, about 97%, about 98%, or about 99% or more sequence identity with the sequences of SEQ ID NOs: 16 to 21. The sequence identity of peptide sequences may be determined using known sequence alignment or comparison software. For example, sequence identity may be determined using the BLASTP program (blast.ncbi.nlm.nih.govBlast.cgi?PAGE=Proteins) with default settings.

The IL-7 may include an IL-7 protein or a fragment thereof, and the fragment is capable of binding to an IL-7 receptor. The term "IL-7 protein" as used herein may be used as a concept including "an IL-7 protein and a fragment thereof capable of binding to an IL-7 receptor." The IL-7 may be obtained from humans, rats, mice, monkeys, cows, or sheep.

Specifically, human IL-7 may have an amino acid sequence represented by SEQ ID NO: 16 (Genbank Accession No. P13232); rat IL-7 may have an amino acid sequence represented by SEQ ID NO: 17 (Genbank Accession No. P56478); mouse IL-7 may have an amino acid sequence represented by SEQ ID NO: 18 (Genbank Accession No. P10168); monkey IL-7 may have an amino acid sequence represented by SEQ ID NO: 19 (Genbank Accession No. NP_001279008); bovine IL-7 may have an amino acid sequence represented by SEQ ID NO: 20 (Genbank Accession No. P26895); and a sheep IL-7 may have an amino acid sequence represented by SEQ ID NO: 21 (Genbank Accession No. Q28540).

In addition, the IL-7 protein or a fragment thereof may include variously modified proteins or peptides, that is, variants. The modification may be performed by substituting, deleting, or adding one or more amino acids in the wild-type IL-7 without altering the function of the IL-7. These various proteins or peptides may have at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, or at least about 99% sequence identity with the wild-type protein.

Typically, a wild-type amino acid residue is substituted with alanine, but substitution may also be performed as conservative amino acid substitutions that have no effect or only a weak effect on the overall protein charge, that is, polarity, or hydrophobicity.

Conservative amino acid substitutions can be found in Table 1 below.

**[Table 1]**

| | |
|---|---|
| Basic | Arginine (Arg, R) |
| | Lysine (Lys, K) |
| | Histidine (His, H) |
| Acidic | Glutamic acid (Glu, E) |
| | Aspartic acid (Asp, D) |
| Uncharged polar | Glutamine (Gln, O) |
| | Asparagine (Asn, N) |
| | Serine (Ser, S) |
| | Threonine (Thr, T) |
| | Tyrosine (Tyr, Y) |
| Non-polar | Phenylalanine (Phe, F) |
| | Tryptophan (Trp, W) |
| | Cysteine (Cys, C) |
| | Glycine (Gly, G) |
| | Alanine (Ala, A) |
| | Valine (Val, V) |
| | Proline (Pro, P) |
| | Methionine (Met, M) |
| | Leucine (Leu, L) |
| | Norleucine |
| | Isoleucine |

For each amino acid, additional conservative substitutions include "homologs" of the amino acid. In particular, a "homolog" refers to an amino acid in which a methylene group (CH₂) is inserted into the beta position side chain of the amino acid. Examples of "homologs" include homophenylalanine, homoarginine, homoserine, and the like, but are not limited thereto.

In a long-acting recombinant IL-7 fusion protein that may be used as one embodiment of the present invention, modified IL-7 may have the following structure:

A - IL-7 Chemical Formula (I)

wherein A is an oligopeptide consisting of one to ten amino acid residues, and the IL-7 is interleukin-7, a polypeptide that may bind to an IL-7 receptor (also known as CD 127) or a polypeptide having the activity of IL-7 or a similar activity thereto.

In the modified IL-7 structure of A-IL-7, the A residue may be directly linked to the N-terminus of IL-7 or linked thereto via a linker to form a fusion protein.

Specifically, the modified IL-7 may have the form of A-IL-7 or A-linker-IL-7.

When the linker is a peptide linker, the linkage may occur at any linkage region. These may be coupled using cross-linking agents known in the art. Examples of cross-linking agents include 1,1-bis(diazoacetyl)-2-phenylethane, glutaraldehyde, N-hydroxysuccinimide esters such as 4-azidosalicylic acid, and imidoesters including disuccinimidyl esters such as 3,3'-dithiobis(succinimidyl propionate) and bifunctional maleimides such as bis-N-maleimido-1,8-octane, but are not limited thereto.

In addition, the linker may be an albumin linker or a peptide linker. The peptide linker may be a peptide of 10 to 20 amino acid residues consisting of Gly and Ser residues.

When the linker is formed of one selected from the group consisting of chemical bonds, the chemical bond may be a disulfide bond, a diamine bond, a sulfide-amine bond, a carboxyl-amine bond, an ester bond, and a covalent bond.

In one embodiment, A may be linked to the N-terminus of IL-7. A is characterized in that it includes one to ten amino acids, wherein the amino acids may be selected from the group consisting of methionine, glycine, and a combination thereof. In one embodiment, when A is a single amino acid residue, it is glycine.

Methionine and glycine do not induce immune responses in humans. Protein therapeutics produced in *E. coli* always include methionine at the N-terminus, but no adverse reactions have been reported. In addition, glycine is widely used as a glycine-serine (GS) linker and is reported not to induce immune responses in commercial products such as dulaglutide (Cell Biophys. 1993 Jan-Jun; 22(103): 189-224).

In one exemplary embodiment, A may be an oligopeptide including one to ten amino acids selected from the group consisting of methionine (Met, M), glycine (Gly, G), and a combination thereof. In one embodiment, A may be an oligopeptide consisting of one to five amino acids. For example, A may be methionine, glycine, methionine-methionine, glycine-glycine, methionine-glycine, glycine-methionine, methionine-methionine-methionine, methionine-methionine-glycine, methionine-glycine-methionine, glycine-methionine-methionine, methionine-glycine-glycine, glycine-methionine-glycine, glycine-glycine-methionine, or glycine-glycine-glycine.

The modified IL-7 may have the structure of A-IL-7 including a polypeptide having the activity of IL-7 or a similar activity thereto and an oligopeptide consisting of one to ten amino acids.

In one embodiment, the modified IL-7 may have an amino acid sequence selected from the group consisting of SEQ ID NOs: 30 to 35. In addition, the modified IL-7 may have a sequence having at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, or at least about 99% sequence identity with the amino acid sequence of SEQ ID NOs: 30, 31, 32, 33, 34 or 35.

A long-acting recombinant IL-7 fusion protein that may be used as one embodiment of the present invention may include:
(a) IL-7 or modified IL-7 of Chemical Formula (I) below:

   A - IL-7 Chemical Formula (I)

   wherein A is an oligopeptide consisting of one to ten amino acid residues; and
(b) a domain that extends the half-life of IL-7 or the modified IL-7.

When the domain extending the half-life of the IL-7 or the modified IL-7 is represented as B, B may be directly linked to the IL-7 or the modified IL-7 or may be linked thereto via a linker. Specifically, the modified IL-7 may have the form of A-IL-7-B, B-A-IL-7, A-IL-7-linker-B, B-linker-A-IL-7, A-linker-IL-7-linker-B, or B-linker-A-IL-7.

When the linker is a peptide linker, the linkage may occur at any linkage region. These may be coupled using cross-linking agents known in the art. Examples of cross-linking agents include 1,1-bis(diazoacetyl)-2-phenylethane, glutaraldehyde, N-hydroxysuccinimide esters such as 4-azidosalicylic acid, and imidoesters including disuccinimidyl esters such as 3,3'-dithiobis(succinimidyl propionate) and bifunctional maleimides such as bis-N-maleimido-1,8-octane, but are not limited thereto.

In addition, the linker may be an albumin linker or a peptide linker. The peptide linker may be a peptide of 10 to 20 amino acid residues consisting of Gly and Ser residues.

When the linker is formed of one selected from the group consisting of chemical bonds, the chemical bond may be a disulfide bond, a diamine bond, a sulfide-amine bond, a carboxyl-amine bond, an ester bond, and a covalent bond.

The domain extending the half-life of the IL-7 or modified IL-7 may be a fusion partner for increasing the *in vivo* half-life of the IL-7 or the modified IL-7, and preferably includes an Fc region of an immunoglobulin or a part thereof, albumin, an albumin-binding polypeptide, Pro/Ala/Ser (PAS), C-terminal peptide of the β-subunit of human chorionic gonadotropin (CTP), polyethylene glycol (PEG), a long unstructured hydrophilic sequence of amino acids (XTEN), hydroxyethyl starch (HES), an albumin-binding small molecule, and a combination thereof.

When the above domain is an Fc region of an immunoglobulin, it may be an Fc region of a modified immunoglobulin. In particular, the Fc region of the modified immunoglobulin may have weakened antibody-dependent cytotoxicity (ADCC) or complement-dependent cytotoxicity (CDC) due to the modification of binding affinity to an Fc receptor and/or complement. The Fc region of the modified immunoglobulin may be selected from the group consisting of IgG1, IgG2, IgG3, IgG4, IgA1, IgA2, IgD, IgE, and a combination thereof. The Fc region of the modified immunoglobulin may be selected from the group consisting of IgG1, IgG2, IgG3, IgD, IgG4, and a combination thereof. Specifically, the Fc region of the modified immunoglobulin may include a hinge region, a CH2 domain, and a CH3 domain from the N-terminus to the C-terminus. In particular, the hinge region may include a human IgD hinge region; the CH2 domain may include some of the amino acid residues of human IgD and some of the amino acid residues of a human IgG4 CH2 domain; and the CH3 domain may include some of the amino acid residues of a human IgG4 CH3 domain.

Meanwhile, two long-acting recombinant IL-7 fusion proteins may form a dimer, and for example, when the domain that extends the half-life of the IL-7 or the modified IL-7 is an Fc region, the Fc regions may be coupled with each other to form a dimer.

The term "Fc region," "Fc fragment," or "Fc" as used herein refers to a protein including the heavy chain constant region 2 (CH2) and the heavy chain constant region 3 (CH3) of an immunoglobulin, but not including the variable regions of the heavy and light chains or the light chain constant region (CL1), and may further include a hinge region of the heavy chain constant regions. In one embodiment, a hybrid Fc or a hybrid Fc fragment thereof may be named "hFc" or "hyFc."

In addition, the term "Fc region variant" as used herein refers to one prepared by substituting some amino acids in an Fc region or combining heterologous Fc regions. An Fc region variant may be prevented from being cleaved at a hinge region. Specifically, amino acid 144 and/or amino acid 145 of SEQ ID NO: 24 may be modified. Preferably, the variant may be one in which the 144th amino acid K is substituted with G or S and the 145th amino acid E is substituted with G or S.

In addition, an Fc region or an Fc region variant of a modified immunoglobulin may be represented by Chemical Formula (II) below:

Chemical formula (II) N'-(Z1)p-Y-Z2-Z3-Z4-C'.

N' is the N-terminus of the polypeptide, and C' is the C-terminus of the polypeptide;
p is an integer of 0 or 1;
Z1 is an amino acid sequence having 5 to 9 consecutive amino acid residues in the N-terminal direction from the amino acid residue at position 98 among the amino acid residues at positions 90 to 98 of SEQ ID NO: 22;
Y is an amino acid sequence having 5 to 64 consecutive amino acid residues in the N-terminal direction from the amino acid residue at position 162 among the amino acid residues at positions 99 to 162 of SEQ ID NO: 22;
Z2 is an amino acid sequence having 4 to 37 consecutive amino acid residues in the C-terminal direction from the amino acid residue at position 163 among the amino acid residues at positions 163 to 199 of SEQ ID NO: 22;
Z3 is an amino acid sequence having 71 to 106 consecutive amino acid residues in the N-terminal direction from the amino acid residue at position 220 among the amino acid residues at positions 115 to 220 of SEQ ID NO: 23; and
Z4 is an amino acid sequence having 80 to 107 consecutive amino acid residues in the C-terminal direction from the amino acid residue at position 221 among the amino acid residues at positions 221 to 327 of SEQ ID NO: 23.

In the present invention, the domain may have an amino acid sequence selected from the group consisting of SEQ ID NOs: 24 to 29.

In addition, the Fc fragment may be a form having native sugar chains, increased sugar chains, or decreased sugar chains compared to the native form, or may be a deglycosylated form. Immunoglobulin Fc sugar chains may be modified by conventional methods such as chemical methods, enzymatic methods, and genetic engineering-based methods using microorganisms. When the sugar chains are removed from the Fc fragment, the binding affinity to the C1q portion of the first complement component C1 is drastically reduced, and ADCC or CDC is reduced or lost, so unnecessary immune responses are not induced *in vivo.* In this regard, an immunoglobulin Fc region in a deglycosylated or aglycosylated form may be more suitable for the purpose of one embodiment as a drug carrier. The term "deglycosylation" as used herein refers to an Fc region from which a sugar has been enzymatically removed from an Fc fragment. In addition, the term "aglycosylation" means that an Fc fragment is produced in a non-glycosylated form by a prokaryotic organism, preferably by *E. coli.*

In addition, the Fc region of the modified immunoglobulin may include an amino acid sequence of SEQ ID NO: 24 (hyFc), SEQ ID NO: 25 (hyFcM1), SEQ ID NO: 26 (hyFcM2), SEQ ID NO: 27 (hyFcM3), or SEQ ID NO: 28 (hyFcM4). In addition, the Fc region of the modified immunoglobulin may include an amino acid sequence of SEQ ID NO: 29 (insoluble mouse Fc).

The Fc region of the modified immunoglobulin may be as described in US Patent No. 7,867,491, and the production of the Fc region of the modified immunoglobulin may be performed by referring to the disclosure of US Patent No. 7,867,491, and the entire contents thereof are incorporated herein by reference.

The long-acting recombinant IL-7 fusion protein may have an amino acid sequence selected from the group consisting of SEQ ID NOs: 36 to 40. In addition, the IL-7 fusion protein may have a sequence having at least about 70%, at least about 75%, at least about 80%, 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, or at least about 99% sequence identity with the amino acid sequence of SEQ ID NOs: 36, 37, 38, 39, or 40.

The long-acting recombinant IL-7 fusion protein of SEQ ID NOs: 36 to 40 may be encoded by a polynucleotide sequence selected from the group consisting of SEQ ID NOs: 50 to 54.

The nucleic acid molecule may further include a signal sequence or leader sequence.

The term "signal sequence" as used herein refers to a fragment that directs the secretion of a biologically active molecular drug and a fusion protein, which is cleaved after being translated in a host cell. In one embodiment, the signal sequence is a polynucleotide encoding an amino acid sequence that initiates the translocation of a protein across the endoplasmic reticulum (ER) membrane. Useful signal sequences in one embodiment include an antibody light chain signal sequence, for example, antibody 14.18 (Gillies et al., J. Immunol. Meth 1989. 125:191-202); an antibody heavy chain signal sequence, for example, the Mouse Oriented Protein of Cell Line 141 (MOPC141) an antibody heavy chain signal sequence (Sakano et al., Nature, 1980. 286: 676-683); and other signal sequences known in the art (see, e.g., Watson et al., Nucleic Acid Research, 1984. 12:5145-5164).

The characteristics of signal peptides are well known in the art, and signal peptides typically have 16 to 30 amino acids, but they may include more or fewer amino acid residues. A typical signal peptide consists of three regions: a basic N-terminal region, a central hydrophobic region, and a more polar C-terminal region.

The central hydrophobic region includes 4 to 12 hydrophobic residues, which anchor the signal sequence through the membrane lipid bilayer during the translocation of the immature polypeptide. After initiation, the signal sequence is cleaved primarily within the lumen of the ER by a cellular enzyme known as a signal peptidase. In particular, the signal sequence may be a secretion signal sequence for tissue plasminogen activation (tPa), a signal sequence of herpes simplex virus glycoprotein D (HSV gD), or a growth hormone. Preferably, a secretion signal sequence used in higher eukaryotic cells, including mammals, may be used. In addition, as a secretion signal sequence, a signal sequence included in wild-type IL-7 may be used or may be used after substitution with a codon with a high expression frequency in a host cell.

An isolated nucleic acid molecule encoding a long-acting recombinant IL-7 fusion protein may be included in an expression vector.

The term "vector" as used herein is understood as a nucleic acid delivery vehicle including a nucleotide sequence that may be introduced into a host cell and recombined and integrated into the genome of the host cell or may independently replicate itself as an episome. Vectors may include linear nucleic acids, plasmids, phagemids, cosmids, RNA vectors, viral vectors, and analogs thereof. Examples of viral vectors include retroviruses, adenoviruses, and adeno-associated viruses, but are not limited thereto.

The term "gene expression" or "expression" of a target protein, as used herein, is understood to refer to the transcription of a DNA sequence, the translation of an mRNA transcript, and the secretion of a fusion protein product or fragment thereof.

The term "host cell" as used herein refers to prokaryotic cells and eukaryotic cells into which a recombinant expression vector may be introduced. The terms "transduced," "transformed," and "transfected," as used herein, refer to the introduction of a nucleic acid (e.g., a vector) into a cell using techniques known in the art.

The term "gene expression" or "expression" of a target protein, as used herein, is understood to refer to the transcription of a DNA sequence, translation of an mRNA transcript, and the secretion of an Fc fusion protein product or an antibody or an antibody fragment thereof.

A useful expression vector may be RcCMV (Invitrogen, Carlsbad) or a variant thereof. The expression vector may include human cytomegalovirus (CMV) to promote the continuous transcription of a target gene in mammalian cells and a polyadenylation signal sequence of a bovine growth hormone to increase the post-transcriptional stability of RNA. In an exemplary embodiment, the expression vector is pAD15, which is a modified form of RcCMV.

An expression vector may be included in an appropriate host cell suitable for the expression and/or secretion of a target protein by transduction or transfection of a DNA sequence of one embodiment.

Examples of appropriate host cells may include immortal hybridoma cells, NS/0 myeloma cells, 293 cells, Chinese hamster ovary (CHO) cells, HeLa cells, human amniotic fluid derived cells (CapT cells), or CV-1 in Origin with SV40 (COS) cells, but are not limited thereto.

The modified IL-7 or long-acting recombinant IL-7 fusion protein may be a material described in US Patent No. 10208099 B2, and the entire contents thereof are incorporated herein by reference.

### Administration route

The pharmaceutical composition according to the present invention may be administered to a subject by direct administration (e.g., topically, by injection into a tissue area, implantation or topical administration) or by a system via an appropriate means (e.g., parenterally or orally). When the composition is administered parenterally, such as intravenously, subcutaneously, ocularly, intraperitoneally, intramuscularly, orally, rectally, intraorbitally, intracerebrally, intracranially, intraspinally, intraventricularly, intrathecally, intracisternally, intracapsularly, intranasally, or by aerosol administration, the composition preferably contains an aqueous or physiologically acceptable suspension of a body fluid or part of a solution thereof. As such, a physiologically acceptable carrier or vehicle may be added to the composition and delivered to a patient, and this does not adversely affect the patient's electrolyte and/or volume balance. Therefore, the physiologically acceptable carrier or vehicle may be a saline solution. The carrier or vehicle may be a pharmaceutically acceptable agent.

### Composition, use, and therapeutic method

In the present invention, the pharmaceutical composition for treating head and neck cancer may be a pharmaceutical composition including an anti-PD1 antibody composition, an HPV vaccine composition, and a long-acting recombinant IL-7 fusion protein composition in separate forms.

The anti-PD1 antibody composition may be a commercially available pembrolizumab or nivolumab formulation. The anti-PD1 antibody may be administered in a dose of about 1 mg to 1000 mg. In one embodiment, the dose of the anti-PD1 antibody may be in the range of about 10 mg to 500 mg, about 50 mg to 500 mg, about 100 mg to 500 mg, about 100 mg to 300 mg, about 150 mg to 300 mg, about 180 mg to 250 mg, about 190 mg to 250 mg, about 185 mg to 225 mg, about 185 mg to 220 mg, about 195 mg to 250 mg, about 195 mg to 225 mg, about 190 mg to 230 mg, about 200 mg to 400 mg, about 200 mg to 300 mg, about 250 mg to 300 mg, about 280 mg to 350 mg, about 300 mg to 500 mg, about 300 mg to 400 mg, about 300 mg to 1000 mg, about 300 mg to 900 mg, about 300 mg to 800 mg, about 300 mg to 700 mg, or about 300 mg to 600 mg. The anti-PD1 antibody may be administered at intervals such as once a week, twice a week, three times a week, four times a week, weekly, every 10 days, once every two weeks, every 20 days, every three weeks, every four weeks, once a month, once every two months, or once every three months. Alternatively, the anti-PD1 antibody may be administered at intervals of 5 days, 10 days, 15 days, 20 days, 30 days, or 40 days. In one embodiment, the anti-PD1 antibody may be pembrolizumab and may be administered in week 1 and week 4.

The anti-PD1 antibody composition may be administered parenterally, preferably intravenously, by injection.

The HPV vaccine composition may be administered in a separate formulation from the anti-PD1 antibody composition. The HPV vaccine composition may be GX-188E.

The composition of GX-188E may be administered parenterally, for example, subcutaneously, intracutaneously, intradermally, subdermally, or intramuscularly, by injection. The composition of GX-188E refers to a composition or formulation including a polynucleotide including SEQ ID NO: 9 or SEQ ID NO: 15 or a variant thereof having about 85% or more, about 86% or more, about 87% or more, about 88% or more, about 89% or more, about 89% or more, about 90% or more, about 91% or about 92% or more, about 93% or more, about 94% or more, about 95% or more, about 96% or more, about 97% or more, about 98% or more, or about 99% or more of sequence identity with SEQ ID NO: 9 or SEQ ID NO: 15. It may be a GX-188E formulation.

The GX-188-containing formulation may be administered in a manner compatible with the dosage formulation and in a therapeutically effective and immunogenic amount. The administered amount depends on the subject to be treated, including, for example, the immune system ability of the subject to cause an immune response and the required degree of immunity. A suitable dosage range is about several hundred micrograms of active ingredient per vaccination, and a preferred range is about 1 µg to 20 mg, for example, about 5 µg to 10 mg. In one aspect, the dose may be in the range of about 0.1 mg to 20 mg, 0.1 mg to 10 mg, or 0.5 mg to 10 mg, particularly in the range of about 0.5 mg to 5 mg. In another aspect, the dose may be in the range of 0.5 mg to 10 mg. In another aspect, the dose may be in the range of 1 mg to 10 mg. In yet another aspect, the dose may be in the range of 1 mg to 5 mg. In yet another aspect, the dose may be in the range of 0.5 mg to 20 mg, 1 mg to 20 mg, 0.5 mg to 5 mg, 1.5 mg to 10 mg, 2 mg to 5 mg, 2.5 mg to 5 mg, 3 mg to 5 mg, or 2 mg to 10 mg. In another embodiment, the dose of the GX-188 may be about 0.5 mg, about 0.6 mg, about 0.7 mg, about 0.8 mg, about 0.9 mg, about 1 mg, about 1.1 mg, about 1.2 mg, about 1.3 mg, about 1.3 mg, about 1.4 mg, about 1.5 mg, about 1.6 mg, about 1.7 mg, about 1.8 mg, about 1.9 mg, about 2 mg, about 2.1 mg, about 1.2 mg, about 1.3 mg, about 1.4 mg, about 1.5 mg, about 1.6 mg, about 1.7 mg, about 1.8 mg, about 1.9 mg, about 2 mg, about 2.1 mg, about 2.2 mg, about 2.3 mg, about 2.4 mg, about 2.5 mg, about 2.6 mg, about 2.7 mg, about 2.8 mg, about 2.9 mg, about 3 mg, about 3.1 mg, about 3.2 mg, about 3.3 mg, about 3.4 mg, about 3.5 mg, about 3.6 mg, about 3.7 mg, about 3.8 mg, about 3.9 mg, about 4 mg, about 4.1 mg, about 4.2 mg, about 4.3 mg, about 4.4 mg, about 4.5 mg, about 4.6 mg, about 4.7 mg, about 4.8 mg, about 4.9 mg, or about 5 mg, and it may be administered approximately once a week, once every two weeks, once every three weeks, or once every four weeks.

The GX-188E formulation may be administered at intervals such as once a week, twice a week, three times a week, four times a week, weekly, every 10 days, once every two weeks, every 20 days, every three weeks, every four weeks, once a month, once every two months, or once every three months. Alternatively, the GX-188E formulation may be administered at intervals of 5 days, 10 days, 15 days, 20 days, 30 days, or 40 days. In one embodiment, the GX-188E formulation may be administered in week 1, week 2, and week 4.

The long-acting recombinant IL-7 fusion protein composition may be administered in a separate formulation from the anti-PD1 antibody composition and the HPV vaccine composition. The long-acting recombinant IL-7 fusion protein composition may be administered parenterally, for example, subcutaneously, intracutaneously, intradermally, subdermally, or intramuscularly, by injection. The long-acting recombinant IL-7 fusion protein may include the amino acid sequence of SEQ ID NO: 39, and for example, it may have the amino acid sequence of SEQ ID NO: 39. The composition means a composition or formulation including a variant thereof having about 85% or more, about 86% or more, about 87% or more, about 88% or more, about 89% or more, about 89% or more, about 90% or more, about 91% or about 92% or more, about 93% or more, about 94% or more, about 95% or more, about 96% or more, about 97% or more, about 98% or more, about 99% or more sequence identity. This may be a GX-I7 formulation.

The long-acting recombinant IL-7 fusion protein-containing formulation may be administered in a manner compatible with a dosage formulation and in a therapeutically effective amount. The formulation may be administered in a pharmaceutically effective amount in preferred ranges of about 10 µg/kg to about 10000 µg/kg, about 30 µg/kg to about 9000 µg/kg, about 50 µg/kg to about 8000 µg/kg, about 60 µg/kg to about 7000 µg/kg, about 70 µg/kg to about 6000 µg/kg, about 80 µg/kg to about 5000 µg/kg, about 90 µg/kg to about 4000 µg/kg or about 100 µg/kg to about 3000 µg/kg.

In another aspect, the formulation may be administered once in a dose of about 100 µg/kg, about 150 µg/kg, about 200 µg/kg, about 250 µg/kg, about 300 µg/kg, about 350 µg/kg, about 400 µg/kg, about 450 µg/kg, about 500 µg/kg, about 550 µg/kg, about 600 µg/kg, about 650 µg/kg, about 700 µg/kg, about 750 µg/kg, about 800 µg/kg, about 850 µg/kg, about 900 µg/kg, about 950 µg/kg, about 1000 µg/kg, about 1050 µg/kg, about 1100 µg/kg, about 1150 µg/kg, about 1200 µg/kg, about 1250 µg/kg, about 1300 µg/kg, about 1350 µg/kg, about 1400 µg/kg, about 1450 µg/kg, about 1500 µg/kg, about 1550 µg/kg, about 1600 µg/kg, about 1650 µg/kg, about 1700 µg/kg, about 1750 µg/kg, about 1800 µg/kg, about 1850 µg/kg, about 1900 µg/kg, about 1950 µg/kg, or about 2000 µg/kg.

The long-acting recombinant IL-7 fusion protein formulation may be administered approximately once a week, once every two weeks, once every three weeks, or once every four weeks. The long-acting recombinant IL-7 fusion protein formulation may be administered at intervals such as once a week, twice a week, three times a week, four times a week, weekly, every 10 days, once every two weeks, every 20 days, every three weeks, every four weeks, once a month, once every two months, or once every three months. Alternatively, the long-acting recombinant IL-7 fusion protein formulation may be administered at intervals of 5 days, 10 days, 15 days, 20 days, 30 days, or 40 days. In one embodiment, the long-acting recombinant IL-7 fusion protein formulation may be administered in week 1, week 2, or week 4. In one embodiment, the long-acting recombinant IL-7 fusion protein formulation may be administered once in week 2.

The pharmaceutical composition for treating head and neck cancer according to the present invention, including an anti-PD1 antibody composition, an HPV vaccine composition, and a long-acting recombinant IL-7 fusion protein composition, may be administered at an appropriate time before cancer resection. For example, the time of the first administration of the pharmaceutical composition may be about 20 weeks before, about 19 weeks before, about 18 weeks before, about 17 weeks before, about 16 weeks before, about 15 weeks before, about 14 weeks before, about 13 weeks before, about 12 weeks before, about 11 weeks before, about 10 weeks before, about 9 weeks before, about 8 weeks before, about 7 weeks before, about 6 weeks before, or about 5 weeks before cancer resection.

In one embodiment, the pharmaceutical composition for treating head and neck cancer may include pembrolizumab as the anti-PD1 antibody, GX-188E as the HPV vaccine, and GX-I7 as the long-acting recombinant IL-7 fusion protein. In a preferred embodiment, pembrolizumab may be administered twice, GX-188E may be administered three times, and GX-I7 may be administered once. For example, pembrolizumab may be administered in an amount of 200 mg intravenously (I.V.) in week 1 and week 4, GX-188E may be administered in an amount of 2 mg intramuscularly (I.M.) in week 1, week 2, and week 4, and GX-I7 may be administered in an amount of up to 1,200 µg/kg intramuscularly (I.M.) in week 2.

In the present invention, the pharmaceutical composition for treating head and neck cancer, including pembrolizumab, may be administered to patients who may undergo cancer resection.

In the present invention, the pharmaceutical composition for treating head and neck cancer, including pembrolizumab, may be administered before cancer resection.

In the present invention, the pharmaceutical composition for treating head and neck cancer, including pembrolizumab, may be administered to patients who have not previously received any other anticancer treatment.

In another embodiment, the pharmaceutical composition for treating head and neck cancer may include nivolumab as the anti-PD1 antibody, GX-188E as the HPV vaccine, and GX-I7 as the long-acting recombinant IL-7 fusion protein. In a preferred embodiment, nivolumab may be administered at two-week intervals, GX-188E may be administered three or more times, and GX-I7 may be administered one or more times. For example, nivolumab may be administered in an amount of 240 mg every two weeks, GX-188E may be administered in an amount of 2 mg in week 1, week 2, week 4, week 7, week 10, week 13, week 19, and week 25, and GX-I7 may be administered in an amount of up to 1,200 µg/kg in week 2, week 10, and week 18.

In addition, the pharmaceutical composition for treating head and neck cancer according to the present invention, including an anti-PD1 antibody composition, an HPV vaccine composition, and a long-acting recombinant IL-7 fusion protein composition, may be administered as a combination therapy at an appropriate time to patients with recurrent and/or metastatic head and neck squamous cell cancer.

In the present invention, the dose and administration interval of each of the drug may be adjusted according to the patient's conditions or drug response level. For example, the dose and administration interval of GX-I7 may be adjusted depending on the patient's conditions or drug response level.

Each of the anti-PD1 antibody composition, the HPV vaccine composition, and the long-acting recombinant IL-7 fusion protein composition may be administered by administration methods other than those described above.

Additional formulations suitable for other administration methods include suppositories and, in some cases, oral, intramuscular, nasal, buccal, sublingual, intraperitoneal, intravaginal, anal, epidural, spinal, and intracranial formulations. For suppositories, traditional binders and carriers may include, for example, polyalkalene glycol or triglycerides, and such suppositories may be formed from mixtures containing an active ingredient in the range from 0.5% to 10% (w/w), preferably from 1% to 2% (w/w). Oral formulations include commonly used excipients such as pharmaceutical grades of mannitol, lactose, starch, magnesium stearate, sodium saccharin, cellulose, magnesium carbonate, and the like. These compositions may be in the forms of solutions, suspensions, tablets, pills, capsules, sustained-release formulations or powder, and may contain an active ingredient in the amount of 10% to 95% (w/w), preferably 25% to 70% (w/w).

In one embodiment, the head and neck cancer may be HPV-positive head and neck cancer. In another embodiment, the head and neck cancer may be head and neck squamous cell cancer. In still another embodiment, the head and neck cancer may be oropharyngeal cancer. In yet another embodiment, the head and neck cancer may be metastatic, recurrent, and/or advanced head and neck cancer. In yet another aspect, the head and neck cancer may be locally advanced. For example, the head and neck cancer may be locally advanced and resectable.

In another aspect, the present invention provides a combination of an anti-PD1 antibody, an HPV vaccine, and a long-acting recombinant IL-7 fusion protein for use in the treatment of head and neck cancer.

In still another aspect, the present invention provides use of a combination of an anti-PD1 antibody, an HPV vaccine, and a long-acting recombinant IL-7 fusion protein in the manufacture of a medicine for treating head and neck cancer.

In yet another aspect, the present invention provides a method of treating head and neck cancer, including administering an anti-PD1 antibody, an HPV vaccine, and a long-acting recombinant IL-7 fusion protein to a subject in need thereof.

In the present invention, the anti-PD1 antibody, the HPV vaccine, and the long-acting recombinant IL-7 fusion protein may be formulated separately and administered simultaneously, separately, or sequentially.

In the present invention, the anti-PD1 antibody, the HPV vaccine, and the long-acting recombinant IL-7 fusion protein may be administered once or several times before cancer resection.

The efficacy of the combination treatment according to the combination therapy of the present invention may be measured by a variety of endpoints commonly used to evaluate cancer treatment, which include tumor regression, tumor weight or size reduction, time to disease progression, overall survival, progression-free survival, overall response rate, duration of response, inhibition of metastatic spread without tumor regression, and positron emission tomography/ computed tomography (PET/CT) imaging, but are not limited thereto.

The terms "combination," "therapeutic combination," "pharmaceutical combination," and "combination" refer to a combination of non-fixed doses, optionally packaged with instructions for combined administration in the cases where the individual therapeutic agents, which are the anti-PD1 antibody, the HPV vaccine, and the long-acting recombinant IL-7 fusion protein, may be administered simultaneously, or where the therapeutic agents may be administered separately within a time interval that allows them to exert their synergistic effects.

The term "simultaneous" administration refers to administering the individual therapeutic agents, which are the anti-PD1 antibody, the HPV vaccine, and the long-acting recombinant IL-7 fusion protein, to a patient in a single operation, for example, administering each of the anti-PD1 antibody, the HPV vaccine, and the long-acting recombinant IL-7 fusion protein independently and substantially at the same time, or separately administering the anti-PD1 antibody, the HPV vaccine, and the long-acting recombinant IL-7 fusion protein within a time interval that allows them to exert their synergistic effects.

The term "separate" administration refers to administering each of the anti-PD1 antibody, the HPV vaccine, and the long-acting recombinant IL-7 fusion protein from a dosage form of a non-fixed dose to a patient simultaneously, substantially simultaneously, or sequentially in any order. There may or may not be a specified time interval for the administration of each of the anti-PD1 antibody, the HPV vaccine, and the long-acting recombinant IL-7 fusion protein.

The term "sequential" administration refers to administering each of the anti-PD1 antibody, the HPV vaccine, and the long-acting recombinant IL-7 fusion protein to a patient in separate operations from non-fixed (separate) dosage forms.

The beneficial action of two therapeutic agents that exert a greater effect than the simple additive effect of the individual therapeutic agents administered alone in a single patient may be calculated, for example, using any suitable method known in the art, such as the Sigmoid-Emax equation (Holford and Scheiner, Clin. Pharmacokinet., 1981, 6: 429-453), the equation of Loewe additivity (Loewe and Muischenk, Arch. Exp. Pathol. Pharmacol., 1926, 114: 313-326), the median-effect equation (Chou and Talalay, Adv. Enzyme Regul., 1984, 22: 27-55), and the Bliss Independence method, or known equivalent methods. By applying each equation to experimental data and preparing corresponding graphs, it is possible to support the hypothesis that the effects of a drug combination are additive, within a biologically relevant additive range, less than additive, or greater than additive.

### Kit

The present invention may be provided as a kit for head and neck cancer treatment, including: an anti-PD1 antibody, an HPV vaccine, and a long-acting recombinant IL-7 fusion protein.

The kit may include an anti-PD1 antibody, an HPV vaccine, and a long-acting recombinant IL-7 fusion protein that are each separately formulated.

The kit may include recommended doses of the anti-PD1 antibody, the HPV vaccine, and the long-acting recombinant IL-7 fusion protein and instructions indicating the recommended administration order and/or recommended doses.

### Examples

Hereinafter, the present invention will be described in more detail through examples. These examples are only for illustrating the present invention, and it will be obvious to those skilled in the art that the scope of the present invention is not to be construed as being limited by these examples.

### 1. Target disease and experimental design

The safety and efficacy of GX-188E, GX-I7, and pembrolizumab combination therapy were explored in patients with resectable locally advanced head and neck squamous cell carcinoma (LA HNSCC) who were HPV-16-positive and/or HPV-18-positive.

The sample size was based on an exact single-stage phase II trial design with major pathological response (MPR) set to be an endpoint.
- Alpha=0.05
- Beta=0.20
- H0=MPR≤5%
- H1=MPR≥35%

Based on the above statistical variables and hypotheses, a clinical trial was conducted with 11 patients as test subjects.

The test subjects were patients aged 19 years or older with LA HNSCC confirmed by histological analysis, and the subjects were HPV-positive patients (here, HPV-positive was confirmed to be positive by p16 immunohistochemistry (IHC) and HPV-16+ and/or HPV-18+ through a nucleic acid test). In addition, the test subjects were those who had no previous history of receiving other anticancer treatment, had one or more measurable lesions according to the Response Evaluation Criteria in Solid Tumors (RECIST) v1.1 criteria, had an Eastern Cooperative Oncology Group (ECOG) performance status of 0 to 1, had a life expectancy of six months or more, had normal organ functions, and consented to provide fresh tumor tissue to enable appropriate levels of use or storage thereof for biomarker analysis, including programmed cell death-ligand 1 (PD-1) analysis staining.

### 2. Combination drug therapy

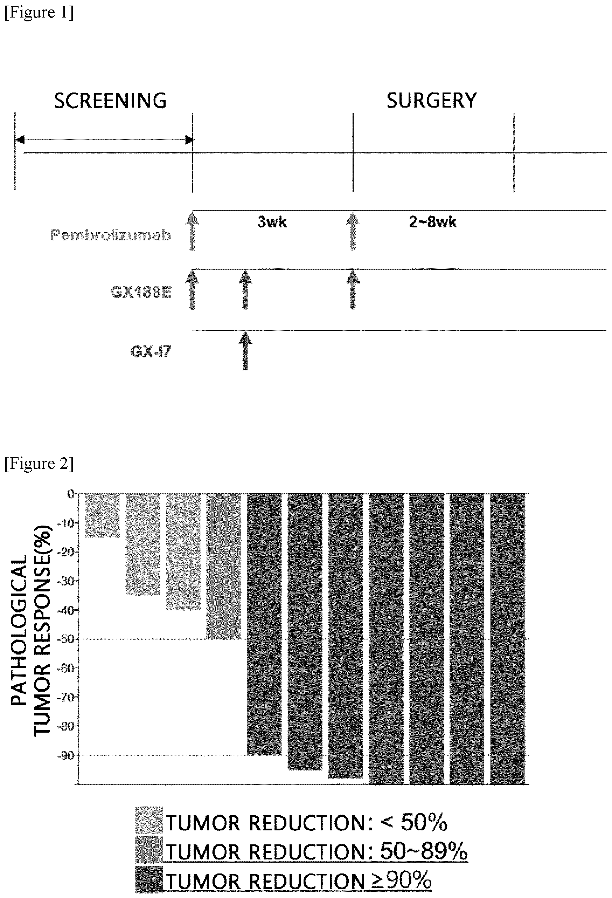

Dosing was carried out according to the schedule shown in FIG. 1. The administration was performed at the doses of pembrolizumab 200 mg I.V. + GX-188E 2 mg I.M. + GX-I7 1200 µg/kg for the specified period.

### Pembrolizumab

Pembrolizumab, used as an example of the anti-PD1 antibody in the present invention, is a potent humanized immunoglobulin G4 (IgG4) monoclonal antibody (mAb) that binds to the PD-1 receptor with high specificity, and it inhibits the interactions of PD-L1 and PD-L2 with PD-1. According to nonclinical *in vitro* data, pembrolizumab has high affinity for PD-1 and strong receptor blocking activity. Pembrolizumab has an acceptable preclinical safety profile and is under clinical development as an intravenously (I.V.) administered immunotherapy for advanced malignancies. Keytruda^{™} Injection (pembrolizumab) has been approved as a therapeutic agent for multiple indications. The product instructions may be referred to obtain detailed information regarding specific indications.

Pembrolizumab is provided as an infusion solution at a dose strength of 100 mg/vial. Pembrolizumab was administered at 200 mg intravenously (I.V.) in week 1 and week 4.

### GX-188E

GX-188E, used as an example of the HPV vaccine in the present invention, is a DNA plasmid (SEQ ID NO: 15) encoding a fusion protein containing the E6 and E7 proteins of HPV-16 and HPV-18 and a human Fms-like tyrosine kinase 3 ligand (hFLT-3L).

2 mg of GX-188E was administered intramuscularly (I.M.) in weeks 1, 2, and 4. GX-188E was administered I.M. in the deltoid muscle or thigh muscle on each scheduled vaccination day. 1 mg of GX-188E was administered to both muscle areas for a total of 2 mg of vaccination per day. As for the GX-188E vaccination site, the deltoid muscle or thigh muscle were alternately used whenever possible, but when necessary, a specific site among the two sites was selected and used, and in this case, the vaccination site was recorded separately.

### GX-I7

GX-I7, used as an example of the long-acting recombinant IL-7 fusion protein in the present invention, is a protein produced by inserting gene expressing rhII,-7-hyFc into a eukaryotic expression vector. The molecular weight of GX-I7 is 104 kDa, and it consists of 400 amino acids, of which 155 amino acids are from IL-7, 30 amino acids are from the IgD hinge, 8 amino acids are from the CH2 domain of IgD, and 207 amino acids are from the IgG4 domain (SEQ ID NO: 39).

GX-I7 was administered intramuscularly (I.M.) at 1200 µg/kg in week 2.

### 3. Cancer resection and post-operative treatment

Cancer resection was performed two to eight weeks after the above-described combination therapy administration was completed.

After surgery, standard post-operative concurrent chemoradiation therapy (PO-CCRTx) based on cisplatin was performed based on pathological findings including high-risk factors (e.g., extra capsule extension [ECE+] or resectional margin involvement [RM+]) or at the doctor's discretion. After completion of PO-CCRTx, as needed, post-operative anticancer adjuvant therapy deemed appropriate was implemented according to the surgical results and the clinician's judgment, and the patient was followed up for five years (tumor evaluation).

### 4. Efficacy evaluation

### (1) Pathological response

Pathological complete response (pCR) and major pathological response (MPR) were confirmed in the 11 patients. pCR was defined as a state where no residual tumor is present, and major pathological response (MPR) was defined as the presence of less than 10% residual tumor.

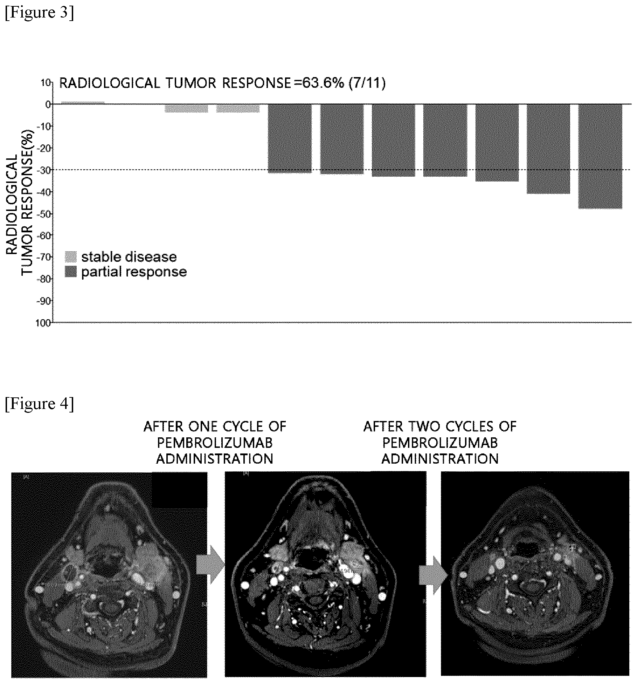

As a result of confirming the pathological response, pCR was achieved in 4 of the 11 patients (36.3%), and MPR was also confirmed in the remaining 7 patients of the 11 patients (63.6%), indicating that the combination drug therapy of the present invention exerted a significant effect in the subject group (see FIGS. 2 and 4).

### (2) Radiological response

Meanwhile, the radiological response of the 11 patients was analyzed using postoperative follow-up images based on the images before the combination drug administration and before the anti-cancer resection. The response evaluation was analyzed based on RECIST 1.1.

As a result of confirming the radiological response, tumor regression of 30% or more was confirmed in 7 of the 11 patients (see FIGS. 3 and 4).

### 5. Safety evaluation

Safety was evaluated in the test subjects. Safety was evaluated by recording, reporting, and analyzing the results of laboratory tests and physical examinations considering the subjects' underlying diseases, adverse reactions, and vital signs. Serious adverse events (SAEs) experienced by the test subjects, such as drug toxicity, were comprehensively evaluated. Investigators assessed the severity of the SAEs based on the National Cancer Institute's Common Terminology Criteria for Adverse Events (CTCAE v4 03).

Major side effects that occurred in the 11 test subjects included fever, increased bilirubin levels, increased creatinine levels, hyperglycemia, and increased alanine transferase, and all patients recovered and the side effects were not associated with a delay in the surgery time (data not shown).

As the specific parts of the present invention have been described in detail above, it is clear to those skilled in the art that these specific descriptions are merely preferred embodiments and do not limit the scope of the present invention. Therefore, the actual scope of the present invention will be defined by the appended claims and their equivalents.

## Claims

1. A pharmaceutical composition for treating head and neck cancer, comprising an anti-programmed death 1 (anti-PD1) antibody, a human papilloma virus (HPV) vaccine, and a long-acting recombinant interleukin-7 (IL-7) fusion protein.

2. The pharmaceutical composition of claim 1, wherein the anti-PD1 antibody is selected from the group consisting of pembrolizumab, nivolumab, cemiplimab, pidilizumab, and a combination thereof.

3. The pharmaceutical composition of claim 1, wherein the HPV vaccine comprises a polynucleotide encoding HPV-16 and HPV-18 antigens.

4. The pharmaceutical composition of claim 3, wherein the HPV vaccine is isolated DNA comprising a polynucleotide encoding the antigens.

5. The pharmaceutical composition of claim 3, wherein the HPV vaccine comprises a polynucleotide of SEQ ID NO: 15 or a functional variant having 85% or more sequence identity with SEQ ID NO: 15.

6. The pharmaceutical composition of claim 3, wherein the HPV vaccine further comprises a nucleic acid sequence encoding a heterologous polypeptide.

7. The pharmaceutical composition of claim 6, wherein the heterologous polypeptide comprises an Fms-like tyrosine kinase 3 ligand ("FLT3L") or a part thereof.

8. The pharmaceutical composition of claim 3, wherein the HPV vaccine further comprises a nucleotide sequence encoding a signal peptide.

9. The pharmaceutical composition of claim 8, wherein the signal peptide is selected from a signal peptide of tissue plasminogen activator (tPA), a signal peptide of herpes simplex virus glycoprotein D (HSV gD), a signal peptide of a growth hormone, and any combination thereof.

10. The pharmaceutical composition of claim 1, wherein the long-acting recombinant IL-7 fusion protein comprises:
(a) IL-7 or modified IL-7 represented by Chemical Formula (I) below:
A - IL-7 Chemical Formula (I),
wherein A is an oligopeptide consisting of one to ten amino acid residues; and
(b) a domain extending the half-life of the IL-7 or the modified IL-7.

11. The pharmaceutical composition of claim 10, wherein the IL-7 has an amino acid sequence selected from the group consisting of SEQ ID NOs: 16 to 21.

12. The pharmaceutical composition of claim 10, wherein the A is an oligopeptide consisting of amino acid residues selected from the group consisting of methionine, glycine, or a combination thereof.

13. The pharmaceutical composition of claim 10, wherein the domain comprises an Fc region of a modified immunoglobulin.

14. The pharmaceutical composition of claim 13, wherein the long-acting recombinant IL-7 fusion protein comprises an amino acid sequence of SEQ ID NO: 39 or a functional variant having 85% or more sequence identity with SEQ ID NO: 39.

15. The pharmaceutical composition of claim 1, wherein the anti-PD-1 antibody is pembrolizumab or nivolumab, and the single dose is 50 mg to 500 mg.

16. The pharmaceutical composition of claim 1, wherein the anti-PD-1 antibody is for intravenous injection.

17. The pharmaceutical composition of claim 1, wherein the single dose of the HPV vaccine is 0.5 mg to 5 mg.

18. The pharmaceutical composition of claim 1, wherein the HPV vaccine is for intramuscular injection.

19. The pharmaceutical composition of claim 1, wherein the single dose of the long-acting recombinant IL-7 fusion protein is 60 µg/kg to 5,000 µg/kg.

20. The pharmaceutical composition of claim 1, wherein the long-acting recombinant IL-7 fusion protein is for intramuscular injection.

21. The pharmaceutical composition of claim 1, wherein the anti-PD1 antibody, the HPV vaccine, and the long-acting recombinant IL-7 fusion protein are each administered once or multiple times.

22. The pharmaceutical composition of claim 1, wherein the anti-PD1 antibody, the HPV vaccine, and the long-acting recombinant IL-7 fusion protein are administered simultaneously, separately, or sequentially.

23. The pharmaceutical composition of claim 1, wherein the anti-PD1 antibody is pembrolizumab, and the pembrolizumab is administered twice, the HPV vaccine is administered three times, and the long-acting recombinant IL-7 fusion protein is administered once.

24. The pharmaceutical composition of claim 1, wherein the anti-PD1 antibody is nivolumab, and the nivolumab is administered at two-week intervals, the HPV vaccine is administered eight times, and the long-acting recombinant IL-7 fusion protein is administered three times.

25. The pharmaceutical composition of claim 1, wherein the pharmaceutical composition is administered before cancer resection.

26. The pharmaceutical composition of claim 1, wherein the head and neck cancer is HPV-positive.

27. The pharmaceutical composition of claim 1, wherein the head and neck cancer is head and neck squamous cell carcinoma.

28. The pharmaceutical composition of claim 1, wherein the head and neck cancer is oropharyngeal cancer.

29. The pharmaceutical composition of claim 1, wherein the head and neck cancer is metastatic, recurrent, and/or advanced.

30. The pharmaceutical composition of claim 1, wherein the head and neck cancer is locally advanced and resectable.

31. A kit for use in the head and neck cancer treatment, comprising an anti-PD 1 antibody, an HPV vaccine, and a long-acting recombinant IL-7 fusion protein.

32. A use of a combination of an anti-PD1 antibody, an HPV vaccine, and a long-acting recombinant IL-7 fusion protein in the manufacture of a medicine for treating head and neck cancer.

33. A combination of an anti-PD1 antibody, an HPV vaccine, and a long-acting recombinant IL-7 fusion protein for use in the treatment of head and neck cancer.

34. A method of treating head and neck cancer, comprising administering an anti-PD1 antibody, an HPV vaccine, and a long-acting recombinant IL-7 fusion protein to a subject in need thereof.

35. The method of claim 34, wherein the anti-PD1 antibody, the HPV vaccine, and the long-acting recombinant IL-7 fusion protein are administered simultaneously, separately, or sequentially.

36. The method of claim 34, wherein the anti-PD1 antibody, the HPV vaccine, and the long-acting recombinant IL-7 fusion protein are administered before cancer resection.
